# EUROPÄISCHE PATENTSCHRIFT

(11) **EP 0 751 963 B1**
(45) Veröffentlichungstag und Bekanntmachung des Hinweises auf die Patenterteilung: **29.12.1997**
(21) Anmeldenummer: 95913084.0
(22) Anmeldetag: 11.03.1995
(51) Int. Cl.: C08F 8/30, C10L 1/22, C08F 222/00, C08F 224/00

(54) **COPOLYMERISATE AUF BASIS VON DIKETENEN, ETHYLENISCH UNGESÄTTIGTEN DICARBONSÄUREN BZW. DICARBONSÄUREDERIVATEN UND ETHYLENISCH UNGESÄTTIGTEN KOHLENWASSERSTOFFEN**
COPOLYMERS BASED ON DIKETENS, ETHYLENICALLY UNSATURATED BICARBOXYLIC ACIDS OR THEIR DERIVATIVES AND ETHYLENICALLY UNSATURATED HYDROCARBONS
COPOLYMERES A BASE DE DICETENES, D'ACIDES DICARBOXYLIQUES INSATURES ETHYLENIQUEMENT OU DE LEURS DERIVES ET D'HYDROCARBURES INSATURES ETHYLENIQUEMENT

(30) Priorität: 24.03.1994 DE 4410198
(43) Veröffentlichungstag der Anmeldung: 08.01.1997
(73) Patentinhaber: BASF AKTIENGESELLSCHAFT, 67056 Ludwigshafen (DE)
(72) Erfinder: FAUL, Dieter, D-67150 Niederkirchen (DE); ROSER, Joachim, D-68165 Mannheim (DE); HARTMANN, Heinrich, D-67117 Limburgerhof (DE); DRALLE-VOSS, Gabriele, D-64297 Darmstadt (DE); OPPENLÄNDER, Knut, D-67061 Ludwigshafen (DE); WENDEROTH, Bernd, D-69488 Birkenau (DE)
(86) Internationale Anmeldenummer: EP9500904
(87) Internationale Veröffentlichungsnummer: WO9525755

(56) Entgegenhaltungen:
- WO-A-90/12821
- FR-A- 2 528 051
- US-A- 4 079 042

## Beschreibung

Die Erfindung betrifft Copolymerisate auf Basis von Diketenen, ethylenisch ungesättigten Dicarbonsäuren bzw. Dicarbonsäurederivaten und ethylenisch ungesättigten Kohlenwasserstoffen, ein Verfahren zu ihrer Herstellung, durch Umsetzung mit NH-, SH- und/ oder OH-funktionellen Verbindungen erhältliche modifizierte Copolymerisate, die Verwendung der gegebenenfalls modifizierten Copolymerisate als Zusatz für Erdölmitteldestillate und diese enthaltende Erdölmitteldestillate.

Mitteldestillate wie Gasöle, Dieselöle oder Heizöle, die durch Destillation aus Erdölen gewonnen werden, haben je nach Herkunft des Rohöls unterschiedliche Gehalte an Paraffinen. Bei tieferen Temperaturen kommt es zur Ausscheidung fester Paraffine (Trübungspunkt oder Cloud Point, CP). Bei weiterer Abkühlung bilden die plättchenförmigen n-Paraffinkristalle eine "Kartenhausstruktur" und das Mitteldestillat stockt, obwohl der überwiegende Teil des Mitteldestillats noch flüssig ist. Durch die ausgefallenen n-Paraffine im Temperaturbereich zwischen Trübungspunkt und Stockpunkt (Pour Point) wird die Fließfähigkeit der Erdöldestillat-Kraftstoffe erheblich beeinträchtigt. Die Paraffine verstopfen Filter und verursachen eine ungleichmäßige oder völlig unterbrochene Kraftstoffzufuhr zu den Verbrennungsaggregaten. Ähnliche Störungen treten bei Heizölen auf.

Es ist seit langem bekannt, daß durch geeignete Zusätze das Kristallwachstum der Paraffine in den Erdölmitteldestillat-Brenn- und Kraftstoffen modifiziert werden kann. Gut wirksame Additive verhindern, daß Mitteldestillate derartige Kartenhaus-Strukturen ausbilden und bei Temperaturen wenige Grad Celsius unterhalb der Temperatur, bei welcher die ersten Paraffinkristalle auskristallisieren, bereits fest werden. Es werden stattdessen feine, gut kristallisierte, separate Paraffinkristalle gebildet, welche Filter in Kraftfahrzeugen und Heizungsanlagen passieren oder zumindest einen für den flüssigen Teil der Mitteldestillate durchlässigen Filterkuchen bilden, so daß ein störungsfreier Betrieb sichergestellt ist.

Als Fließverbesserer werden seit langem Ethylen-Vinylcarboxylat-Copolymere eingesetzt, wie sie z.B. aus der US-A-3 048 479 und US-A-3 627 838 bekannt sind.

Ein Nachteil dieser Additive beruht darin, daß die ausgefallenen Paraffinkristalle aufgrund ihrer gegenüber dem flüssigen Teil höheren Dichte dazu neigen, sich beim Lagern mehr und mehr am Boden des Behälters abzusetzen. Dadurch bildet sich im oberen Behälterteil eine homogene paraffinarme Phase und am Boden eine zweiphasige paraffinreiche Schicht. Da sowohl in Fahrzeugtanks als auch in Lager- oder Liefertanks der Mineralölhändler der Abzug des Mitteldestillates meist wenig oberhalb des Behälterbodens erfolgt, besteht die Gefahr, daß die hohe Konzentration an festen Faraffinen zu Verstopfungen von Filtern und Dosiereinrichtungen führt. Diese Gefahr wird um so größer, je weiter die Lagertemperatur die Ausscheidungstemperatur der Paraffine unterschreitet, da die ausgeschiedene Paraffinmenge mit sinkender Temperatur ansteigt.

Bei den Paraffinkristallmodifikatoren, den sog. Fließverbesserern oder Paraffindispergatoren, handelt es sich im allgemeinen um Polymere, die durch Co-Kristallisation (Interaktion) das Kristallwachstum der n-Paraffine verändern und die Fließeigenschaften des Mitteldestillats bei niedrigen Temperaturen verbessern. Die Wirksamkeit der Fließverbesserer wird nach DIN EN 116 indirekt durch Messung des "Cold Filter Plugging Point" (CFPP) ausgedrückt.

Aus der DE-A-23 42 300 sind Copolymerisate aus Maleinsäureanhydrid (MSA) und Diketen bekannt. Diese Copolymerisate bzw. deren vollständig oder teilweise hydrolisierte Form werden als geeignete Produkte zur Herstellung von oberflächenaktiven Verbindungen, Haarsprayzusammensetzungen, Glasreinigern, Textilhilfsmitteln oder Bindemitteln zur Verfestigung von Vliesstoffen und Papier beschrieben.

In der DE-A-2 531 194 und DE-A-2 531 195 werden MSA/Diketen-Copolymere bzw. MSA/Diketen/Vinylether-Copolymerisate bzw. deren Umsetzungsprodukte mit Alkoholen mit 1 - 18 Kohlenstoffatomen, Polyethylenglykolmonoalkylethern sowie mit Gemischen aus beiden beschrieben. Diese werden insbesondere in Textilhilfsmitteln und Haarkosmetika eingesetzt.

In der DE-A-3 913 127 werden Copolymerisate auf Basis von MSA/Diketen und Acrylsäure beschrieben. Diese Polymerisate eignen sich nach ihrer Isolierung oder ggf. nach Solvolyse als Waschmitteladditive.

Es bestand die Aufgabe, Copolymerisate zur Verfugung zu stellen, die die Fließfahigkeit von Erdölmitteldestillaten bei tiefer Temperatur gewährleisten, indem sie eine solche Dispergierwirkung haben, daß ein Absetzen ausgeschiedener Paraffine verzögert oder verhindert wird. Die Fließverbesserer sollen ihre Wirkung unabhängig von der Zusammensetzung der Erdölmitteldestillate entfalten.

Demgemäß wurden Copolymerisate aus
a) 1 bis 65 Mol-% mindestens eines Diketens der allgemeinen Formel I 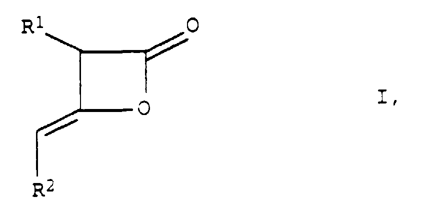 worin R¹ und R² unabhängig voneinander Wasserstoff oder C₁-C₃₀-Alkyl bedeuten,
b) 30 bis 70 Mol-% mindestens einer ethylenisch ungesättigten Dicarbonsäure oder eines Dicarbonsäurederivates,
c) 0,5 bis 60 Mol-% mindestens eines ethylenisch ungesättigten Kohlenwasserstoffes und
d) bis 20 Mol-% mindestens eines weiteren ethylenisch ungesättigten Monomeren
gefunden.

Gegenstand der Erfindung sind außerdem als Paraffindispergatoren geeignete modifizierte Copolymerisate, die durch Umsetzung der vorgenannten Copolymerisate mit NH-, SH- und/oder OH-funktionellen Verbindungen erhältlich sind, die Verwendung der gegebenenfalls modifizierten Copolymerisate als Zusatz für Erdolmitteldestillate sowie diese Copolymerisate enthaltende Erdölmitteldestillate.

Gegenstand der Erfindung ist außerdem ein Verfahren zur Herstellung von Copolymerisaten, wobei
a) 1 bis 65 Mol-% mindestens eines Diketens der allgemeinen Formel I 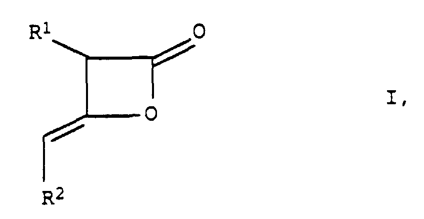 worin R¹ und R² unabhängig voneinander Wasserstoff oder C₁-C₃₀-Alkyl bedeuten,
b) 30 bis 70 Mol-% mindestens einer ethylenisch ungesättigten Dicarbonsäure oder eines Dicarbonsäurederivates,
c) 0,5 bis 60 Mol-% mindestens eines ethylenisch ungesättigten Kohlenwasserstoffes und
d) bis 20 Mol-% mindestens eines weiteren ethylenisch ungesättigten Monomeren
in Gegenwart eines Radikale bildenden Polymerisationsinitiators umgesetzt werden.

Die erfindungsgemäßen Copolymerisate enthalten als wesentlichen Bestandteil einpolymerisiert 1 bis 65 Mol-%, vorzugsweise 10 bis 50 Mol-%, mindestens eines Diketens der allgemeinen Formel I (Komponente a)).

Ein Beispiel für ein substituiertes Diketen ist das unter der Bezeichnung Basoplast® von der BASF Aktiengesellschaft vertriebene Distearyldiketen, bei dem R¹ und R² jeweils 14 bis 16 Kohlenstoffatome aufweisen. Erfindungsgemäß wird bevorzugt das unsubstituierte Diketen (R¹ und R² gleich Wasserstoff) eingesetzt.

Die erfindungsgemäßen Copolymerisate enthalten 30 bis 70 Mol-%, vorzugsweise 40 bis 60 Mol-% mindestens einer ethylenisch ungesättigten Dicarbonsäure oder eines Dicarbonsäurederivates (Komponente b)) einpolymerisiert.

Als Komponente b) eignen sich beispielsweise monoethylenisch ungesättigte Dicarbonsäureanhydride mit 4 bis 8 C-Atomen, z.B. Maleinsäureanhydrid, Itaconsäureanhydrid, Mesaconsäureanhydrid, Citraconsäureanhydrid und Methylenmalonsäureanhydrid. Von den genannten Anhydriden werden Maleinsäureanhydrid und Itaconsäureanhydrid bevorzugt eingesetzt, wobei Maleinsäureanhydrid ganz besonders bevorzugt ist.

Geeignet sind des weiteren beispielsweise Fumarsäure, Maleinsäure, Itaconsäure, Mesaconsäure, Citraconsäure, Methylenmalonsäure sowie deren Ester.

Die erfindungsgemäß bevorzugten ethylenisch ungesättigten Dicarbonsäuren oder Dicarbonsäurederivate lassen sich in der allgemeinen Formel II zusammenfassen, 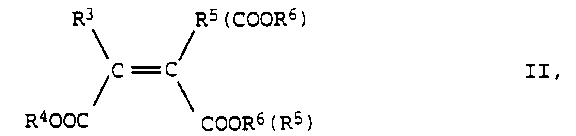 worin R³ bis R⁶ unabhängig voneinander Wasserstoff oder einen gegebenenfalls Heteroatome enthaltenden C₁-C₂₂-Alkylrest bedeuten, wobei es sich im Falle von cis-Dicarbonsäuren der Formel II (R⁴=R⁶=H) auch um deren Säureanhydride handeln kann.

Die erfindungsgemäßen Copolymerisate enthalten als weiteren wesentlichen Bestandteil 0,5 bis 60 Mol-%, vorzugsweise 5 bis 40 Mol-% mindestens eines ethylenisch ungesättigten Kohlenwasserstoffes (Komponente c)). Als Komponente c) werden bevorzugt ethylenisch ungesättigte Kohlenwasserstoffe der allgemeinen Formel III 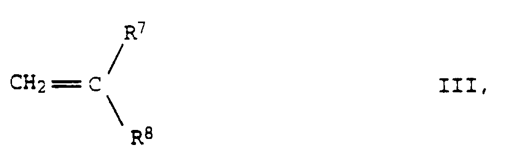 worin R⁷ Wasserstoff oder einen C₁-C₁₀-Alkylrest und R⁸ einen Alkyl-, Alkenyl- oder Arylrest bedeutet, eingesetzt. Geeignete Verbindungen c) sind beispielsweise α-Olefine wie 1-Buten, 1-Hexen, 1-Octen, 1-Decen, 1-Dodecen, 1-Tetradecen, 1-Hexadecen, 1-Octadecen oder Gemische von 1-Alkenen mit 20 - 24 oder 24 - 28 C-Atomen oder längerkettige Polyethylenabkömmlinge mit endständiger CC-Doppelbindung. Auch endständig ungesättigte polymere Propen-, Buten- und Isobuten-Derivate sind geeignet. Weiterhin kommen Aryl-substituierte Olefine wie z.B. Styrol in Frage.

Die erfindungsgemäßen Copolymerisate können darüber hinaus noch bis 20 Mol-% mindestens eines weiteren ethylenisch ungesättigten Monomeren enthalten (Komponente d)). Beispiele für Monomere d) sind Acrylsäure, Methacrylsäure, daraus abgeleitete Ester sowie Vinylether und Vinylester.

Die Polymerisation der Komponenten a), b), c) und gegebenenfalls d) erfolgt im allgemeinen in an sich bekannter Weise in inerten organischen Lösungsmitteln unter radikalischen Bedingungen (vgl. DE-A-23 42 300 bzw. DE-A-25 31 135). Geeignet sind insbesondere Lösungsmittel, welche praktisch nicht in die Polymerisation eingreifen und nicht mit den Monomeren reagieren. Solche Lösungsmittel, die alleine oder in Mischung verwendet werden können, sind beispielsweise Aceton, Methylethylketon, Diethylketon, Cyclohexanon, Tetrahydrofuran, Dioxan, Ethylacetat, Propionsäureethylester, aromatische Kohlenwasserstoffe, wie Benzol, Toluol, Xylol, Cumol, Tetralin, Solvent Naphtha (z.B. Solvesso®150), aliphatische Kohlenwasserstoffe, wie Pentan, Hexan, Heptan, Octan, Isooctan, Cyclohexan, Decalin, Shellsol®D70, halogenierte aliphatische Kohlenwasserstoffe, wie Dichlormethan, Dichlorethan, Trichlorethan.

Solvesso®150 ist die Bezeichnung der Fa. EXXON Chemical GmbH für eine aromatische Lösungsmittelfraktion vom Siedeintervall 187 bis 203°C. Es enthält ca. 99 % Aromaten. Shellsol®D70 ist die Bezeichnung der Fa. Shell für ein entaromatisiertes, aliphatisches Kohlenwasserstoffgemisch vom Siedeintervall 195 bis 245°C.

Vorzugsweise verwendet man als Lösungsmittel Aceton, Methylethylketon, Toluol, Xylol, Tetralin, Dekalin, Solvent Naphtha (z.B. Solvesso® 150) oder Shellsol®D70.

Die Copolymerisation der Komponenten a), b), c) und ggf. d) erfolgt in der Regel in Gegenwart von Verbindungen, die unter den Polymerisationsbedingungen in Radikale zerfallen. Geeignete Polymerisationsinitiatoren sind beispielsweise Wasserstoffperoxid, organische Peroxide und Hydroperoxide, Azoverbindungen und Peroxodisulfate. Die Polymerisation kann auch durch Einwirkung energiereicher Strahlung oder durch Bestrahlung des Reaktionsgemisches in Gegenwart eines Photoinitiators, wie beispielsweise Benzoin, vorgenommen werden.

Die Initiatoren sollen vorzugsweise bei den gewählten Polymerisationstemperaturen eine Halbwertszeit von < 3 Std. besitzen. Vorzugsweise werden als Polymerisationsinitiatoren tert.-Butylperpivalat, Dilaurylperoxid, tert.-Butyl-per-2-ethylhexanoat (tButylperoctoat), tert.-Butylperbenzoat, Dicumpylperoxid, Di-tert.-butylperoxid und 2,2'-Azobis(2-methylpropionitril), alleine oder in Mischung, eingesetzt. Durch die Mitverwendung von Redox-Coinitiatoren, wie beispielsweise Benzoin oder Dimethylanilin sowie von organisch löslichen Komplexen oder Salzen von Schwermetallen, wie Kupfer, Kobalt, Mangan, Eisen, Nickel, Chrom, können die Halbwertzeiten der genannten Peroxide verringert werden.

Die in Radikale zerfallenden Polymerisationsinitatoren werden in üblichen Mengen eingesetzt, z.B. 0,1 bis 5,0 Gew.-%, bezogen auf die bei der Polymerisation verwendeten Mengen an Monomeren.

Die Copolymerisation kann ggf. in Gegenwart von üblichen Reglern, wie Mercaptoethanol, Mercaptopropanol, Mercaptoessigsäure, Mercaptopropionsäure, Thiomilchsäure, n-Butylmercaptan, Butylmercaptan, Octylmercaptan und Dodecylmercaptan durchgeführt werden. Weitere geeignete Regler sind Aldehyde, wie Acetaldehyd, Propionaldehyd und Butyraldehyd sowie Ameisensäure.

Die Polymerisation wird vorzugsweise in Rührkesseln durchgeführt, die beispielsweise mit einem Anker-, Blatt- oder Impellerrührer ausgestattet sind. Die Copolymerisation kann beispielsweise als Lösungs-, Fällungs- oder Suspensionspolymerisation durchgeführt werden. Bei der Fällungs- und Suspensionspolymerisation kann es vorteilhaft sein, zusätzlich in Gegenwart von Schutzkolloiden zu polymerisieren. Geeignete Schutzkolloide sind beispielsweise Copolymerisate aus Maleinsäureanhydrid und Vinylalkylethern, die 1 bis 20 Kohlenstoffatome in der Alkylgruppe enthalten, oder Copolymerisate aus Maleinsäureanhydrid und Olefinen mit 8 bis 20 Kohlenstoffatomen sowie deren Monoester mit C₁₀- bis C₂₀-Alkoholen oder Monoamide mit C₁₀- bis C₂₀-Aminen. Außerdem eignen sich Polyalkylvinylether, deren Alkylgruppe 1 bis 20 Kohlenstoffatome enthält, z.B. Polymethyl-, Polyethyl- und Polyisobutylvinylether. Falls bei der Copolymerisation ein Schutzkolloid eingesetzt wird, so betragen die wirksamen Mengen 0,05 bis 4,0 Gew.-%, bezogen auf die zu polymerisierenden Monomeren a), b), c) und ggf. d).

Die Konzentration der Monomeren in den inerten organischen Lösungen beträgt im allgemeinen 5 bis 80, vorzugsweise 15 bis 60 Gew.-%. Die Polymerisationstemperatur liegt in der Regel im Bereich von 40 bis 160°C, vorzugsweise 50 bis 150°C.

Nach Beendigung der Polymerisation können die Copolymerisate isoliert werden, z.B. durch Abdestillieren des bei der Polymerisation verwendeten Lösungsmittels bzw. durch Ausfällen der Polymeren mit einem geeigneten Lösungsmittel. Die Copolymerisate verbleiben dann als pulverförmiger Rückstand.

Das mittlere Molekulargewicht (Gewichtsmittel) der erfindungsgemäßen Copolymerisate beträgt im allgemeinen 300 bis 50 000, vorzugsweise 500 bis 10 000.

Die Copolymerisate können als solche in Erdölmitteldestillaten eingesetzt werden. Vorzugsweise werden diese jedoch durch polymeranaloge Umsetzungen modifiziert.

Die erfindungsgemäßen Copolymerisate enthalten als reaktionsfähige Gruppen insbesondere Carboxyl-, Anhydrid-, Ester- und 4-Ring-Lacton-Struktureinheiten. Vorteilhaft sind neben der einfachen Hydrolyse mit Wasser auch Umsetzungen mit NH-, SH- und OH-funktionellen Verbindungen möglich.

Geeignete Reaktionspartner sind z.B. Alkohole, Phenole, Mercaptane, Oxime, Imine, primäre und sekundäre Amine. Die Umsetzung der Copolymerisate kann hierbei in dem organischen Lösungsmittel erfolgen, in dem auch die Copolymerisation vorgenommen wurde oder man destilliert die verwendeten Lösungsmittel zunächst ab und führt dann die entsprechenden Umsetzungen durch.

Als Alkohole können insbesondere verzweigte oder geradkettige C₁-bis C₃₀-Alkylalkohole oder Alkenylalkohole, vorzugsweise C₁₂-C₃₀-Alkyl- oder Alkenylalkohole, eingesetzt werden, z.B. i-Tridecanol, Stearylalkohol, Talgfettalkohol, Behenylalkohol. Es können auch Alkoxilate von Alkoholen, Aminen, Amiden oder Carbonsäuren eingesetzt werden, z.B. Alkoxilierungsprodukte von Distearylamin, Oleylamin, Ditalgfettamin, hydriertem Ditalgfettamin, Dikokosfettamin, Aminopropylstearylamin, Stearylalkohol, i-Tridecanol, i-Tridecylamin, Behenylalkohol.

Als Amine können primäre oder sekundäre Alkylamine, Polyetheramine oder Polyamine eingesetzt werden.

Als Beispiele für primäre oder sekundäre Amine seien i-Tridecylamin, Stearylamin, Kokosfettamin, Distearylamin, Ditalgfettamin, Dioleylamin, Dikokosfettamin genannt. Es können auch Polyamine eingesetzt werden z.B. Aminopropylstearylamin, Aminopropyllaurylamin, N,N-Dimethylpropylendiamin, N,N-Dimethyldipropylentriamin. Als Aminkomponente können auch aminopropylierte Alkohole, aminierte Alkoxilate oder aminopropylierte Alkoxilate eingesetzt werden, z.B. Aminopropylstearylalkohol, Aminopropylethoxystearylamin, Fettalkyl-polyethylenglykol-amin (aminierte Lutensol®-Marken der BASF Aktiengesellschaft).

Besonders geeignete modifizierte Copolymerisate sind durch Umsetzung der erfindungsgemäßen Copolymerisate mit NH- und/oder OH-funktionellen Verbindungen der allgemeinen Formei IV erhältlich, 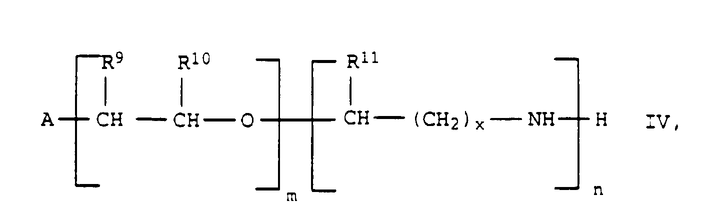 worin
m=0 bis 100, n=0 bis 5 und x=0 bis 5 sein können,
R⁹, R¹⁰ und R¹¹ unabhängig voneinander Wasserstoff oder einen C₁-C₅-Alkylrest bedeuten,
A für NR¹²R¹³ oder einen C₂-C₃₀-Alkoxirest steht, worin mindestens einer der Substituenten R¹² oder R¹¹ von Wasserstoff verschieden ist und einen C₁-C₃₀-Alkyl- oder Alkenylrest oder einen Polyetherrest 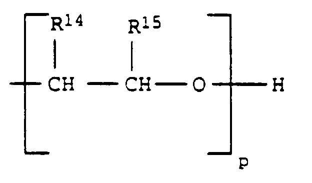 mit R¹⁴ und R¹⁵ unabhängig voneinander Wasserstoff oder C₁-C₅-Alkyl und p 1 bis 100 bedeutet.

Der R¹² und/oder R¹³ bildende C₁-C₃₀-Alkyl- oder Alkenylrest ist vorzugsweise ein C₃-C₃₀- und besonders bevorzugt ein C₁₄-C₂₄-Alkylrest, der mehrfach ungesättigt und gegebenenfalls verzweigt sein kann.

In einer bevorzugten Ausführungsform der modifizierten Copolymerisate ist m oder n gleich 0.

Beispiele für Verbindungen der Formel IV, für die m=0 ist, sind Distearylamin, Ditalgfettamin, Dioleylamin, Dikokosfettamin, oder auch Aminopropylstearylamin und Aminopropyllaurylamin.

Beispiele für weitere Verbindungen der Formel IV, für die n=0 und A=NR¹²R¹³ ist, sind die Alkoxilierungsprodukte von Distearylamin, Oleylamin, Ditalgfettamin, hydriertem Talgfettamin, Dikokosfettamin, Aminopropylstearylamin. Für n=O ist m vorzugsweise 1 bis 10. Besonders bevorzugt sind alkoxiliertes Stearylamin und alkoxyliertes Distearylamin, z.B. Hydroxyethyldistearylamin.

Die Umsetzung der erfindungsgemäßen Copolymerisate mit Alkoholen und Aminen bzw. deren Derivaten erfolgt in Substanz oder in solchen Lösungsmitteln, die im allgemeinen selbst keine aciden Wasserstoffatome enthalten. Es können die gleichen Lösungsmittel wie bei der Polymerisation eingesetzt werden. Die Reaktion muß nicht vollständig ablaufen. Die während der Umsetzung gebildeten Amid- und Ester-Gruppen sowie die Abnahme der Konzentration des 4-Ringlactons können IR-spektroskopisch verfolgt werden. Gleichzeitig wird eine Abnahme der Säurezahl [mg KOH/g] beobachtet.

Die Alkohol- oder Aminkomponente wird bezogen auf ihre OH- oder NH-Äquivalente üblicherweise in Mengen von 0,1 bis 3 Val pro Val einpolymerisierter Dicarbonsäure(derivat)-Menge eingesetzt. Bevorzugt sind Mengen von 0,5 bis 2 Val NH oder OH pro Dicarbonsäure(derivat)-Einheit.

Als geeignete Paraffindispergatoren für Mitteldestillate haben sich vor allem die Umsetzungsprodukte der erfindungsgemäßen Copolymerisate mit Fettalkoholen, wie Stearylalkohol, Talgfettalkohol oder Behenylalkohol bzw. alkoxilierten Fettalkoholen (z.B. Lutensol® AT-Marken der BASF AG) sowie mit primären und sekundären Alkylaminen erwiesen.

Die erfindungsgemäßen gegebenenfalls modifizierten Copolymerisate finden Verwendung als Zusatz für Erdölmitteldestillate, worunter Petroleum, Heizöl und Dieselkraftstoffe mit einer Siedetemperatur von etwa 150 bis 400°C verstanden werden. Die Copolymerisate können den Mitteldestillaten direkt, bevorzugt aber als 20 bis 70 gew.-%ige Lösung zugesetzt werden. Geeignete Lösungsmittel sind aliphatische oder aromatische Lösungsmittel wie Xylol oder deren Gemische, weiterhin hochsiedende Aromatengemische, sowie Mitteldestillate. Die Menge an Copolymerisaten in den Erdölmitteldestillaten beträgt in der Regel 10 bis 10 000, vorzugsweise 20 bis 5000 und besonders bevorzugt 50 bis 1000 ppm. Je nach Verwendungszweck können die Mitteldestillate noch weitere Additive wie z.B. Fließverbesserer, Dispergatoren, Anti-Schaum-Mittel, Korrosionsschutzmittel, Antioxidantien, Demulgatoren, Schmierfähigkeitsverbesserer, Leitfähigkeitsverbesserer und/oder Farbstoffe enthalten.

Üblicherweise enthalten solche Mitteldestillate bereits bekannte Fließverbesserer, die in der Patentliteratur eingehend beschrieben sind, z.B. in DE-A-19 14 756 und EP-A-486 836 (Ethylen-Vinylester-Copolymerisate und deren Mischung mit anderen Copolymeren), EP-A-214 876 (a-Olefin-Maleinsäureanhydridester-Copolymerisate) oder EP-A-155 807 (Alkylfumarat-Vinylacetat-Copolymere).

Es kommen jedoch auch gleichermaßen Copolymerisate in Betracht, die neben Ethylen und Vinylestern oder Acrylestern noch weitere Comonomere enthalten. Das Molekulargewicht dieser Fließverbesserer beträgt in der Regel 500 bis 5000, vorzugsweise 1000 bis 3000.

Die erfindungsgemaßen Copolymerisate bewirken in Mitteldestillaten unabhängig von deren Herkunft eine deutliche Verbesserung der Fließeigenschaften in der Kälte, indem sie ausgeschiedene Paraffinkristalle wirksam in der Schwebe halten, so daß es nicht zu Verstopfungen von Filtern und Leitungen durch abgesetztes Paraffin kommt. Sie weisen eine gute Breitenwirksamkeit auf und bewirken so, daß die ausgeschiedenen Paraffinkristalle in unterschiedlichen Mitteldestillaten sehr gut dispergiert werden.

### Beispiele

Die Prozentangaben in den Beispielen beziehen sich jeweils auf das Gewicht. Die K-Werte wurden nach H. Fikentscher, Cellulosechemie, Band 13, Seiten 58 bis 64 und 71 bis 74 (1932) bestimmt.
A) Herstellung der Copolymerisate
Zur Herstellung der Copolymerisate wurde ein Reaktor aus Glas, der mit Rührer und drei Zuläufen versehen war, verwendet und unter Stickstoff gearbeitet. Ein Zulauf war beheizbar und für die Lösung von Maleinsäureanhydrid in Solvesso® 150 auf 65°C eingestellt. Im IR-Spektrum der Copolymerisate zeigten sich jeweils charakteristische Banden bei 1840 cm⁻¹ (Lacton, Anhydrid) und 1780 cm⁻¹ (Anhydrid).
A)
   Im Reaktor wurden 27,9 g eines α-Olefins der Fraktion C₂₀-C₂₄ (Gulftene® 20-24; Warenzeichen der Fa. Chevron) und 52 g Solvesso®150 vorgelegt und auf 90°C erhitzt. Anschließend wurden innerhalb von 3 Stunden 45,6 g Maleinsäureanhydrid, gelöst in 40 g Solvesso® 150, und 34 g Diketen zugefügt. Parallel hierzu wurde innerhalb von 3,5 Stunden eine Lösung von 2,72 g tert.-Butylperoctoat in 40 g Solvesso® 150 zugefügt. Nach beendeter Initiatorzugabe wurde das Reaktionsgemisch noch 2 Stunden bei 100°C gerührt. Es wurde eine gelbe, viskose Lösung mit einem Feststoffgehalt von 28,3 % erhalten. Nach Einengen der Lösung im Vakuum wurde das Polymer als gelbes Pulver mit einem K-Wert von 22,5 (2%ig in Xylol) erhalten.
A2)
   Im Reaktor wurden 97,8 g eines α-Olefins der Fraktion C₂₀-C₂₄ (Gulftene® 20-24)und 32 g Solvesso® 150 vorgelegt und auf 90°C erhitzt. Anschließend wurden innerhalb von 3 Stunden 40,8 g Maleinsäureanhydrid, gelöst in 35 g Solvesso® 150, und 7,8 g Diketen, gelöst in 35 g Solvesso® 150, zugefügt. Parallel hierzu wurde innerhalb von 3,5 Stunden eine Lösung von 3,6 g tert.-Butylperoctoat in 38 g Solvesso® 150 zugefügt. Nach beendeter Initiatorzugabe wurde das Reaktionsgemisch noch 2 Stunden bei 100°C gerührt. Es wurde eine gelbe, viskose Lösung mit einem Feststoffgehalt von 47,7 % erhalten. Nach Einengen der Lösung im Vakuum wurde das Polymer als gelbes Pulver mit einem K-Wert von 12,5 (5%ig in Xylol) erhalten.
A3)
   Im Reaktor wurden 37,3 g eines α-Olefins der Fraktion C₂₄-C₂₈ (Gulftene® 24-28)und 50 g Solvesso® 150 vorgelegt und auf 90°C erhitzt. Anschließend wurden innerhalb von 3 Stunden 50,2 g Maleinsäureanhydrid, gelöst in 35 g Solvesso® 150, und 37,7 g Diketen zugefügt. Parallel hierzu wurde innerhalb von 3,5 Stunden eine Lösung von 3,14 g tert.-Butylperoctoat in 40 g Solvesso® 150 zugefügt. Nach beendeter Initiatorzugabe wurde das Reaktionsgemisch noch 2 Stunden bei 100°C gerührt. Es wurde eine gelbe, viskose Lösung mit einem Feststoffgehalt von 27,3 % erhalten. Nach Einengen der Lösung im Vakuum wurde das Polymer als gelbes Pulver mit einem K-Wert von 17,5 (2%ig in Xylol) erhalten.
A4)
   Im Reaktor wurden 145,8 g eines α-Olefins der Fraktion C₁₄-C₂₈ (Gulftene® 24-28)und 50 g Solvesso® 150 vorgelegt und auf 90°C erhitzt. Anschließend wurden innerhalb von 3 Stunden 49 g Maleinsäureanhydrid, gelöst in 30 g Solvesso® 150, und 9,4 g Diketen, gelöst in 35 g Solvesso® 150, zugefügt. Parallel hierzu wurde innerhalb von 3,5 Stunden eine Lösung von 3,6 g tert.-Butylperoctoat in 55 g Solvesso® 150 zugefügt. Nach beendeter Initiatorzugabe wurde das Reaktionsgemisch noch 2 Stunden bei 100°C gerührt. Es wurde eine gelbe, viskose Lösung mit einem Feststoffgehalt von 52,6 % erhalten. Nach Einengen der Lösung im Vakuum wurde das Polymer als gelbes Pulver mit einem K-Wert von 13,5 (5%ig in Xylol) erhalten.
A5)
   Im Reaktor wurden 78,0 g eines α-Olefins basierend auf Polyisobuten mit einem gewichtsmittleren Molekulargewicht 1000 (PIB [1000]; Glissopal® ES 3250 der BASF Aktiengesellschaft) und 52 g Solvesso® 150 vorgelegt und die Lösung auf 90°C erhitzt. Anschließend wurden innerhalb von 3 Stunden 38,2 g Maleinsäureanhydrid, gelöst in 35 g Solvesso® 150, und 29,0 g Diketen zugefügt. Zusammen mit der Zugabe von Monomeren dosierte man innerhalb von 3,5 Stunden eine Lösung von 3,6 g ^{t}Butylperoctoat in 55 g Solvesso® 150 zu. Nach Beendigung der Initiatorzugabe wurde das Reaktionsgemisch noch 2 Stunden bei 100°C gerührt. Es wurde eine gelbe, viskose Lösung mit einem Feststoffgehalt von 43,3 % erhalten. Nach dem Einengen der Lösung im Vakuum ließ sich das Polymer in Form eines gelbes Pulvers isolieren. Das Polymer hatte einen K-Wert von 18,5 (5%ig in Xylol).
A7)
   Im Reaktor wurden 30,4 g Maleinsäureanhydrid und 65 g Solvesso® 150 vorgelegt und auf 90°C erhitzt. Anschließend wurden innerhalb von 3 Stunden 6,5 g Styrol, gelöst in 25 g Solvesso® 150, und 23 g Diketen, gelöst in 25 g Solvesso® 150, zugefügt. Zusammen mit der Zugabe von Monomeren dosierte man innerhalb von 3,5 Stunden eine Lösung von 1,5 g ^{t}Butylperoctoat in 25 g Solvesso® 150 zu. Nach Beendigung der Initiatorzugabe wurde das Reaktionsgemisch noch 2 Stunden bei 100°C gerührt. Es wurde eine gelbe, ölige Suspension vom Feststoffgehalt von 26,7 % erhalten. Nach dem Absaugen und Trocknen erhielt man das Polymer in Form eines schwach gelben Pulvers.
A8)
   Im Reaktor wurden 29,4 g Maleinsäureanhydrid und 60 g Solvesso® 150 vorgelegt und auf 90°C erhitzt. Anschließend wurden innerhalb von 3 Stunden 25 g Styrol, gelöst in 25 g Solvesso® 150, und 5,6 g Diketen, gelöst in 25 g Solvesso® 150 zugefügt. Zusammen mit der Zugabe von Monomeren dosierte man innerhalb von 3,5 Stunden eine Lösung von 1,5 g ^{t}Butylperoctoat in 25 g Solvesso® 150 zu. Nach beendeter Initiatorzugabe wurde das Reaktionsgemisch noch 2 Stunden bei 100°C gerührt. Es wurde eine gelbe, ölige Suspension mit einem Feststoffgehalt von 31,8 % erhalten. Nach dem Absaugen und Trocknen erhielt man das Polymer in Form eines schwach gelben Pulvers.
A9)
   Im Reaktor wurde eine Lösung von 70,9 g Distearyldiketen, 16,3 g MSA und 9,9 g eines α-Olefins der Fraktion C₂₄-C₂₈ (Gulftene® 24-28) in 24 g Solvesso® 150 vorgelegt und auf 90°C aufgeheizt. Innerhalb von 3,5 h wurde eine Lösung von 4,85 g Butylperoctoat in 25 g Solvesso® 150 zugegeben. Nach beendetem Zulauf wurde noch 2 h bei 95°C nachpolymerisiert.
   Die so erhaltene viskose Lösung hatte einen Feststoffgehalt von 66,9 % und wurde ohne weitere Aufarbeitung direkt umgesetzt.
A10)
   Im Reaktor wurde eine Lösung von 78,8 g Distearyldiketen, 18,15 g MSA und 2,5 g Lutonal® A 50 (Schutzkolloid der BASF Aktiengesellschaft) in 17 g Solvesso® 150 vorgelegt und auf 90°C aufgeheizt. Innerhalb von 3,5 h wurde eine Lösung von 5,0 g ^{t}Butylperoctoat in 20 g Solvesso® 150 zugegeben. Parallel hierzu wurden innerhalb von 2,5 h 3,85 g Styrol, gelöst in 10 g Solvesso® 150, zudosiert. Nach beendetem Zulauf wurde noch 2 h bei 100°C nachpolymerisiert. Die so erhaltene gelb-braune Suspension hatte einen Feststoffgehalt von 70,4 % und wurde ohne weitere Aufarbeitung direkt umgesetzt.
A11)
   Im Reaktor wurde eine Lösung von 130,3 g eines Diesters der Fumarsäure, hergestellt durch Veresterung mit einem C_{12/14}-Alkyl-alkohol (ALFOL® 1412 H der Firma Condea), in 37 g o-Xylol vorgelegt und auf 90°C aufgeheizt. Dann wurden die folgenden Zuläufe in den jeweils angegebenen Zeiten parallel zudosiert: 29,6 g C_{20/24-}Olefin (Gulftene® 20-24), gelöst in 21 g Solvesso® 150 (3 h), 33,6 g Diketen gelöst in 134 g Solvesso® 150 (3 h) und 9,7 g ^{t}Butylperoctoat, gelöst in 40 g Solvesso® 150 (3,5 h). Nach beendetem Zulauf wurde noch 2 h bei 100°C nachpolymerisiert. Die so erhaltene gelbe ölige Polymerlösung hatte einen Feststoffgehalt von 41,2 % und wurde ohne weitere Aufarbeitung direkt umgesetzt.

B) Umsetzung der erfindungsgemäßen Polymere A) mit ethoxilierten Fettalkoholen
Allgemeine Vorschrift:
Die in Tabelle 1 angegebene Menge einer Polymerlösung A) wurde mit der entsprechenden Menge des ethoxilierten Stearylalkohols (Lutensol® AT-Marken der BASF Aktiengesellschaft) bzw. des Alkylalkohols versetzt und nach dem Verdünnen mit der angeführten Menge Solvesso® 150 5 Stunden auf 100°C erhitzt. Das Reaktionsende konnte IR-spektroskopisch verfolgt werden. Die Carbonylschwingung des 4-Ring-Lactons bzw. der Carbonsäureanhydrid-Gruppierung bei 1840 cm⁻¹ nahmen ab zugunsten einer Esterbande bei 1730 cm⁻¹.

**Tabelle 1**

| Beispiel | Polymer | Alkohol-Komponente | Solvesso® 150 |
|---|---|---|---|
| B1 | 18,5 g A3 | 57,6 g Lutensol AT 25 | 30 g |
| B2 | 11,6 g A3 | 61,4 g Lutensol AT 50 | 55 g |
| B3 | 13,5 g A5 | 43,0 g Lutensol AT 25 | 40 g |
| B4 | 9,0 g A5 | 49,0 g Lutensol AT 50 | 45 g |
| B5 | 6,7 g A2 | 49,0 g Lutensol AT 50 | 48 g |
| B6 | 7,9 g A6 | 7,7 g Lutensol AT 11 | 9 g |
| B7 | 7,9 g A6 | 14,4 g Lutensol AT 25 | 16 g |
| B8 | 12,2 g A3 | 13 g 2-Ethylhexanol | 35 g |
| B9 | 36,6 g A6 | 21,5 g Oleylalkohol | 25 g |

C) Umsetzung der erfindungsgemäßen Polymere A) mit Aminen
Allgemeine Vorschrift:
Die in Tabelle 2 angegebene Menge einer Polymerlösung A) wurde mit der entsprechenden Menge der Aminkomponente versetzt und nach dem Verdünnen mit der angeführten Menge Solvesso® 150 5 bis 8 Stunden auf 100°C erhitzt. Das Reaktionsende konnte IR-spektroskopisch verfolgt werden. Die Carbonylschwingung des 4-Ring-Lactons bzw. der Carbonsäureanhydrid-Gruppierung bei 1840 cm⁻¹ nahmen ab zugunsten einer Amidbande bei 1650 bzw. 1680 cm⁻¹. Gleichzeitig wurde eine Abnahme der Säurezahl beobachtet. 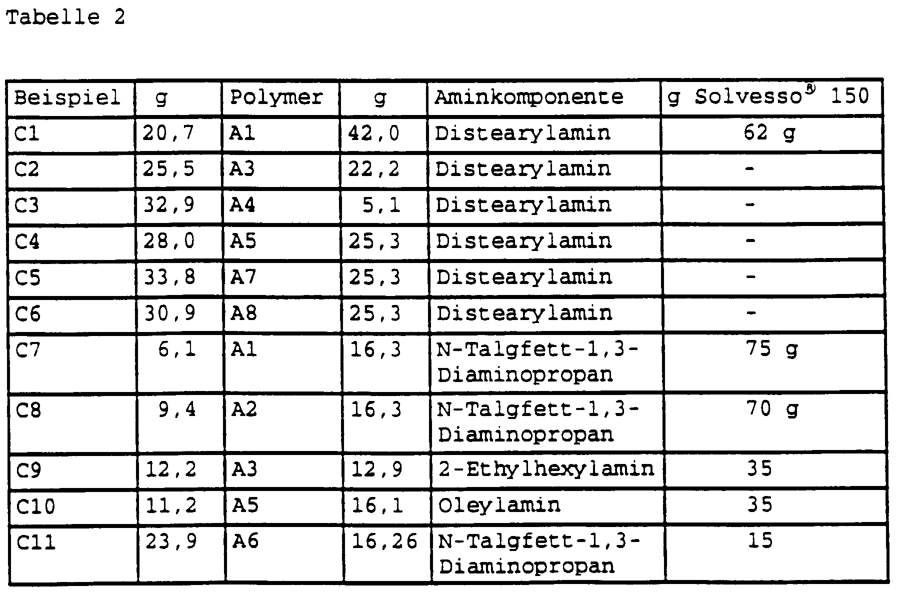 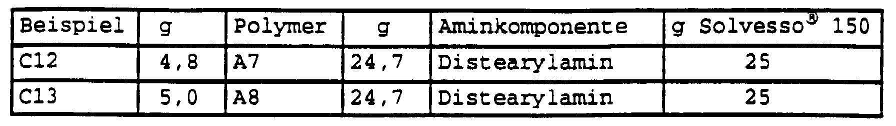
D. Verwendungsbeispiele
Die erfindungsgemäßen Copolymerisate wurden in einer Reihe von Erdölmitteldestillaten geprüft. Es handelte sich dabei um Dieselkraftstoffe in handelsüblicher deutscher Raffineriequalität; sie werden als DK 1, DK 2 und DK 3 bezeichnet:

| | DK 1 | DK 2 | DK 3 |
|---|---|---|---|
| Trübungspunkt CP (°C) | -8 | -7 | -5 |
| CFPP (°C) | -10 | -9 | -8 |
| Dichte b. 20°C (g/ml) | 0.831 | 0.826 | 0.838 |
| Siedeanfang (°C) | 175 | 172 | 167 |
| 20 % Siedepunkt (°C) | 223 | 217 | 221 |
| 90 % Siedepunkt (°C) | 314 | 321 | 328 |
| Siedeende (°C) | 352 | 356 | 361 |

### 2.1 Beschreibung der Testmethoden

Die Mitteldestillate wurden mit den in den Tabellen angegebenen Mengen der erfindungsgemäßen Copolymerisate B1 bis B7, C1 bis C6 und/oder einem bekannten Fließverbesserer F1 auf Basis eines Ethylen-Vinylpropionat-Copolymerisats mit ca. 40 Gew.-% Vinylpropionat und einem mittleren Molekulargewicht von 2500 bei 40°C unter Rühren additiviert und anschließend auf Raumtemperatur abgekühlt.

### Test 1

Die additivierten Mitteldestillate wurden in 100 ml-Messzylindern für 20 Stunden in einem Kälteschrank bei -13°C gelagert. Anschließend wurden visuell Volumen und Aussehen sowohl der sedimentierten Paraffinphase als auch der darüber stehenden Ölphase bestimmt und beurteilt. Zusätzlich wurde von jeder Probe der "Cold Filter Plugging Point" (CFPP) nach DIN EN 116 gemessen.

Die Ergebnisse sind in Tabelle 3 aufgeführt. Es wird ersichtlich, daß durch die erfindungsgemäßen Copolymerisate B1 bis B7 in Gegenwart des Fließverbesserers F1 eine sehr gute Dispergierung der n-Paraffine erzielt wird. Im Gegensatz dazu hat der Fließverbesserer F1 allein keine dispergierende Wirkung.

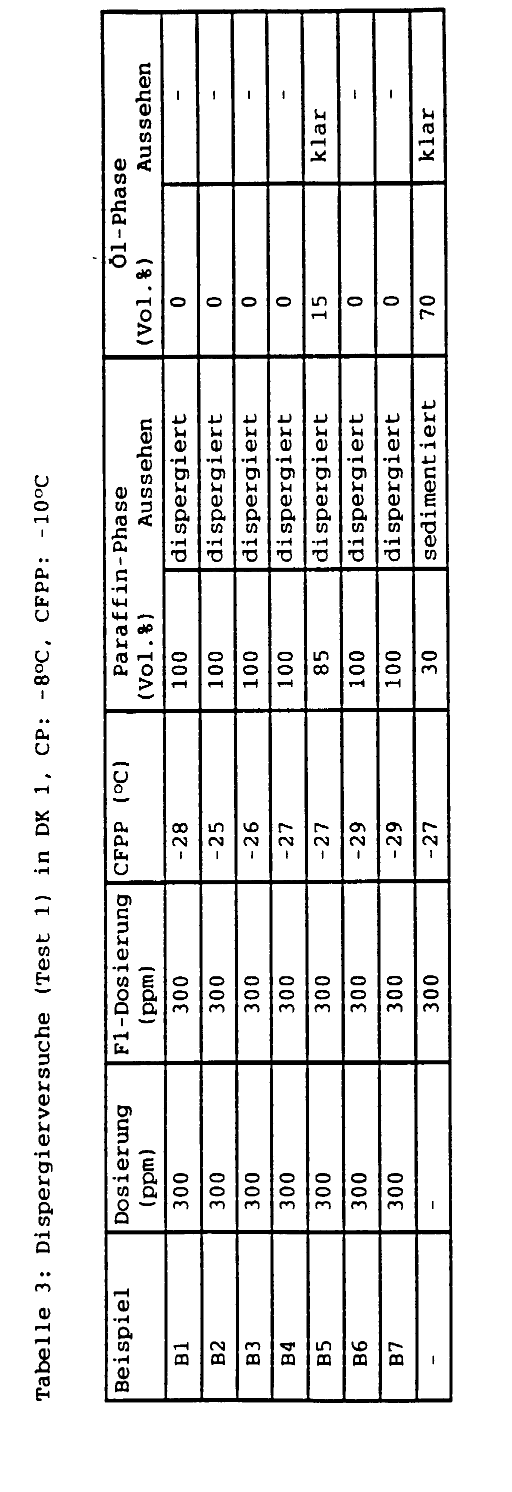

### Test 2

Die additivierten Mitteldestillate wurden in 500 ml-Glaszylindern in einem Kältebad von Raumtempertur auf -13°C abgekühlt und 20 Stunden bei dieser Temperatur gelagert. Anschließend wurde visuell Menge und Aussehen der Paraffinphase bestimmt und beurteilt. Von der bei -13°C abgetrennten 20 Vol-%-Bodenphase wurde von jeder Probe der "Cold Filter Plugging Point" (CFPP) nach DIN EN 116 und der Cloud Point (CP) nach ASTM D 2500 ermittelt.

In den Tabellen 4 bis 6 sind die Ergebnisse aufgeführt. Ergänzend zur visuellen Beurteilung zeigt die gute Übereinstimmung des CP der 20 Vol-%-Bodenphase mit dem CP des jeweiligen Mitteldestillates, daß praktisch eine vollständige Dispergierung der n-Paraffine erreicht wurde.

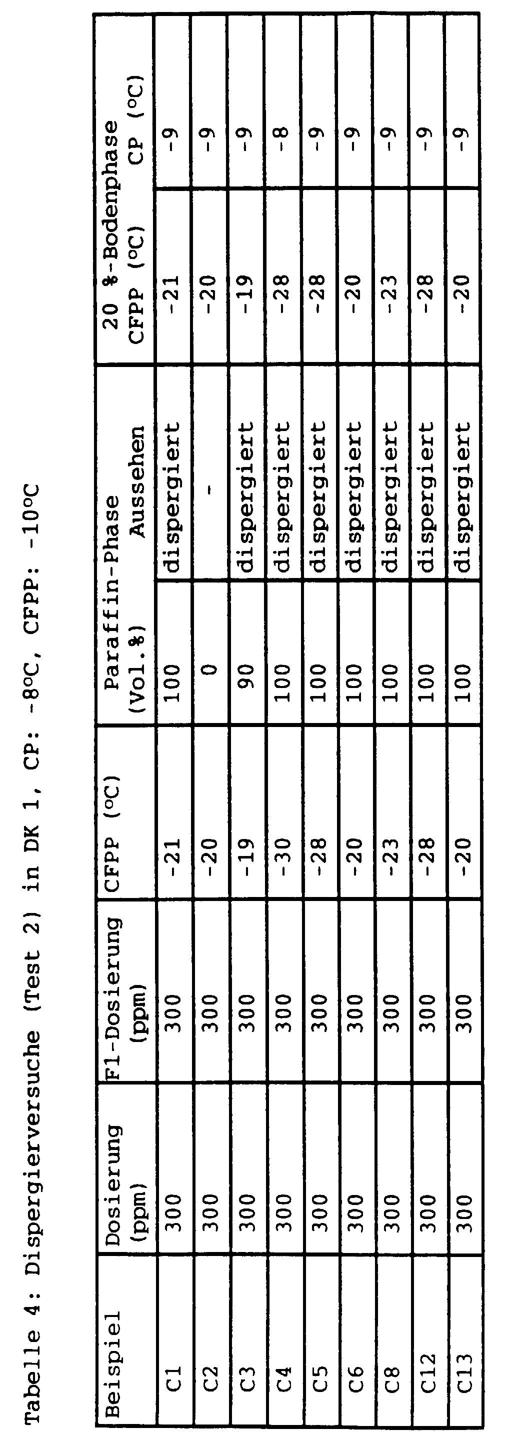

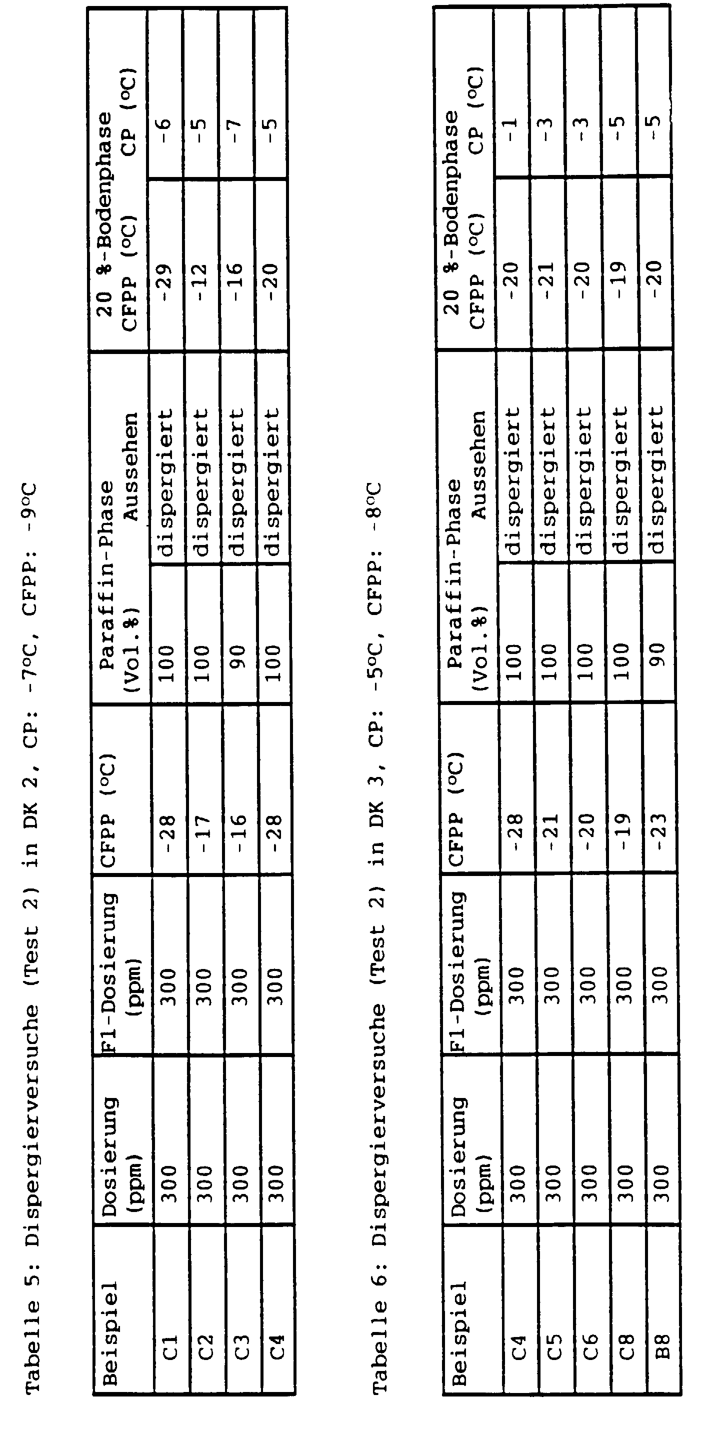

## Patentansprüche

1. Copolymerisate aus
a) 1 bis 65 Mol-% mindestens eines Diketens der allgemeinen Formel I, 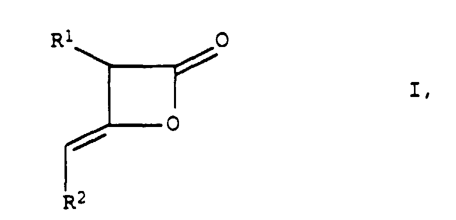 worin R¹ und R² unabhängig voneinander Wasserstoff oder C₁-C₃₀-Alkyl bedeuten,
b) 30 bis 70 Mol-% mindestens einer ethylenisch ungesättigten Dicarbonsäure oder eines Dicarbonsäurederivates,
c) 0,5 bis 60 Mol-% mindestens eines ethylenisch ungesättigten Kohlenwasserstoffes und
d) bis 20 Mol-% mindestens eines weiteren ethylenisch ungesättigten Monomeren.

2. Copolymerisate nach Anspruch 1, dadurch gekennzeichnet, daß R¹ und R² Wasserstoff sind.

3. Copolymerisate nach Anspruch 1 oder 2, dadurch gekennzeichnet, daß die ethylenisch ungesättigten Dicarbonsäuren oder Dicarbonsäurederivate b) der allgemeinen Formel II 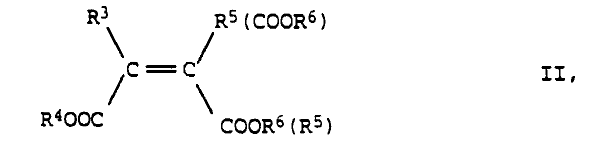 worin R³ bis R⁶ unabhängig voneinander Wasserstoff oder einen gegebenenfalls Heteroatome enthaltenden C₁-C₂₂-Alkylrest bedeuten, entsprechen, oder die Säureanhydride der jeweiligen cis-Dicarbonsäuren der Formel II sind.

4. Copolymerisate nach Anspruch 3, dadurch gekennzeichnet, daß R³ und R⁵ Wasserstoff sind.

5. Copolymerisate nach einem der Ansprüche 1 bis 4, dadurch gekennzeichnet, daß die ethylenisch ungesättigten Kohlenwasserstoffe der allgemeinen Formel III, 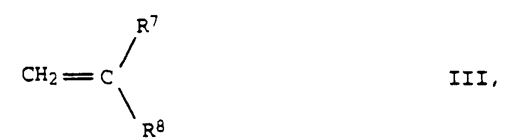 worin R⁷ Wasserstoff oder einen C₁-C₁₀-Alkylrest und R⁸ einen Alkyl-, Alkenyl- oder Arylrest bedeutet, entsprechen.

6. Als Paraffindispergatoren geeignete modifizierte Copolymerisate, erhältlich durch Umsetzung von Copolymerisaten nach einem der Ansprüche 1 bis 5 mit NH-, SH- und/oder OH-funktionellen Verbindungen.

7. Modifizierte Copolymerisate nach Anspruch 6, dadurch gekennzeichnet, daß als NH- und/oder OH-funktionelle Verbindung eine Verbindung der allgemeinen Formel IV, 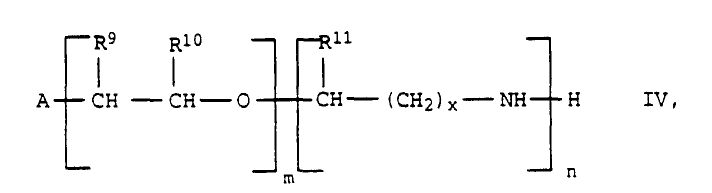 worin
m = 0 bis 100, n = 0 bis 5 und x = 0 bis 5 sein können,
R⁹, R¹⁰ und R¹¹ unabhängig voneinander Wasserstoff oder einen C₁-C₅-Alkylrest bedeuten,
A für NR¹²R¹³ oder einen C₂-C₃₀-Alkoxirest steht, worin mindestens einer der Substituenten R¹² oder R¹³ von Wasserstoff verschieden ist und einen C₁-C₃₀-Alkyl- oder Alkenylrest oder einen Polyetherrest 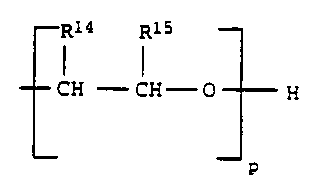 mit R¹⁴ und R¹⁵ Wasserstoff oder C₁-C₅-Alkyl und p 1 bis 100 bedeutet, verwendet wird.

8. Verwendung der Copolymerisate gemäß einem der Ansprüche 1 bis 7 als Zusatz für Erdölmitteldestillate.

9. Erdölmitteldestillate, enthaltend Copolymerisate gemäß einem der Ansprüche 1 bis 7.

10. Verfahren zur Herstellung von Copolymerisaten gemäß Anspruch 1, dadurch gekennzeichnet, daß
a) 1 bis 65 Mol-% mindestens eines Diketens der allgemeinen Formel I, 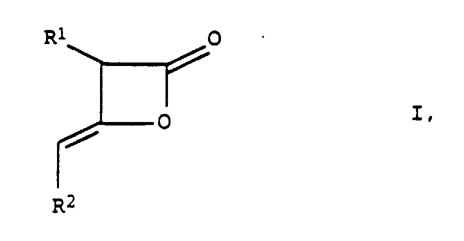 worin R¹ und R² unabhängig voneinander Wasserstoff oder C₁-C₃₀-Alkyl bedeuten,
b) 30 bis 70 Mol-% mindestens einer ethylenisch ungesättigten Dicarbonsäure oder eines Dicarbonsäurederivates,
c) 0,5 bis 60 Mol-% mindestens eines ethylenisch ungesättigten Kohlenwasserstoffes und
d) bis 20 Mol-% mindestens eines weiteren ethylenisch ungesättigten Monomeren
in Gegenwart eines Radikale bildenden Polymerisationsinitiators umgesetzt werden.

## Claims

1. A copolymer of
a) from 1 to 65 mol% of at least one diketene of formula I 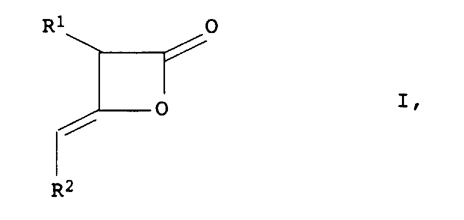 where R¹ and R², independently of one another, are each hydrogen or C₁-C₃₀-alkyl,
b) from 30 to 70 mol% of at least one ethylenically unsaturated dicarboxylic acid or one dicarboxylic acid derivative,
c) from 0.5 to 60 mol% of at least one ethylenically unsaturated hydrocarbon and
d) up to 20 mol% of at least one further ethylenically unsaturated monomer.

2. A copolymer as claimed in claim 1, wherein R¹ and R² are each hydrogen.

3. A copolymer as claimed in claim 1 or 2, wherein the ethylenically unsaturated dicarboxylic acid or dicarboxylic acid derivative b) is of the formula II 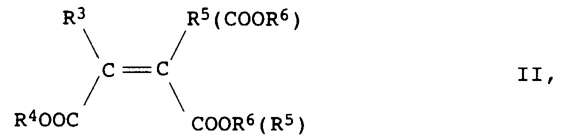 where R³ to R⁶, independently of one another, are each hydrogen or a C₁-C₂₂-alkyl radical which may contain hetero atoms, or are the anhydride of the particular cis-dicarboxylic acid of the formula II.

4. A copolymer as claimed in claim 3, wherein R³ and R⁵ are each hydrogen.

5. A copolymer as claimed in any of claims 1 to 4, wherein the ethylenically unsaturated hydrocarbons are of the formula III 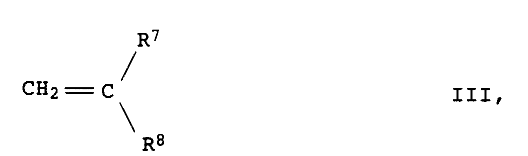 where R⁷ is hydrogen or C₁-C₁₀-alkyl and R⁸ is alkyl, alkenyl or aryl.

6. A modified copolymer which is suitable as a paraffin dispersant and is obtainable by reacting a copolymer as claimed in any of claims 1 to 5 with NH-, SH- or OH-functional compounds.

7. A modified copolymer as claimed in claim 6, wherein the NH- or OH-functional compound used is a compound of the formula IV 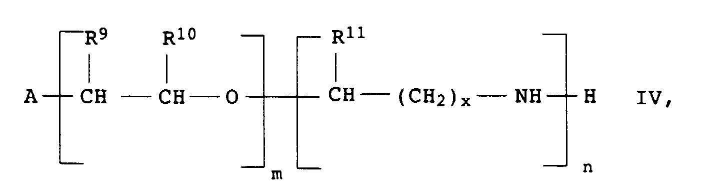 where
m may be from 0 to 100, n from 0 to 5 and x from 0 to 5,
R⁹, R¹⁰ and R¹¹, independently of one another, are each hydrogen or C₁-C₅-alkyl, and
A is NR¹²R¹³ or C₂-C₃₀-alkoxy,
and at least one of the substituents R¹² or R¹³ is not hydrogen and is C₁-C₃₀-alkyl or alkenyl or a polyether radical 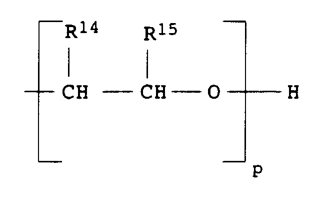 where R¹⁴ and R¹⁵ are each hydrogen or C₁-C₅-alkyl and p is from 1 to 100.

8. Use of a copolymer as claimed in any of claims 1 to 7 as an additive for mineral oil middle distillates.

9. A mineral oil middle distillate containing a copolymer as claimed in any of claims 1 to 7.

10. A process for the preparation of a copolymer as claimed in claim 1, wherein
a) from 1 to 65 mol% of at least one diketene of formula I 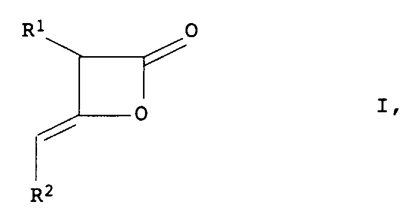 where R¹ and R², independently of one another, are each hydrogen or C₁-C₃₀-alkyl,
b) from 30 to 70 mol% of at least one ethylenically unsaturated dicarboxylic acid or one dicarboxylic acid derivative,
c) from 0.5 to 60 mol% of at least one ethylenically unsaturated hydrocarbon and
d) up to 20 mol% of at least one further ethylenically unsaturated monomer,
are reacted in the presence of a free radical polymerization initiator.

## Revendications

1. Copolymères composés de
a) 1 à 65 % en moles d'au moins un dicétène de formule générale I, 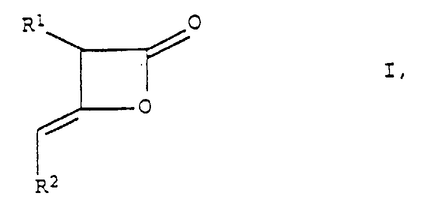 où R¹ et R² sont mis indépendamment l'un de l'autre, pour un atome d'hydrogène ou un groupement alkyle en C₁-C₃₀,
b)30 à 70 % en moles d'au moins un acide dicarboxylique à insaturation éthylénique ou d'un dérivé d'acide dicarboxylique,
c) 0,5 à 60 % en moles d'au moins un hydrocarbure à insaturation éthylénique et
d) jusqu'à 20% en moles d'au moins un autre monomère à insaturation éthylénique.

2. Copolymères selon la revendication 1, caractérisés en ce que R¹ et R² sont des atomes d'hydrogène.

3. Copolymères selon la revendication 1 ou 2, caractérisés en ce que les acides dicarboxyliques à insaturation éthylénique ou les dérivés d'acides dicarboxyliques b) correspondent à la formule générale II 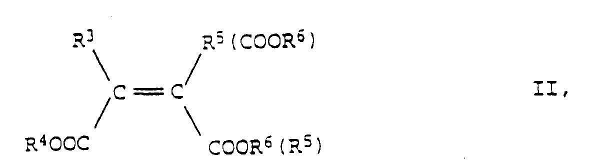 dans laquelle R³ à R⁶ sont mis, indépendamment les uns des autres, pour un atome d'hydrogène ou pour un reste alkyle en C₁-C₂₂ contenant éventuellement des hétéroatomes, ou bien sont les anhydrides de chacun des acides dicarboxyliques cis de formule II.

4. Copolymères selon la revendication 3, caractérisés en ce que R³ et R⁵ sont des atomes d'hydrogène.

5. Copolymères selon l'une quelconque des revendications 1 à 4, caractérisés en ce que les hydrocarbures à insaturation éthylénique correspondent à la formule générale III, 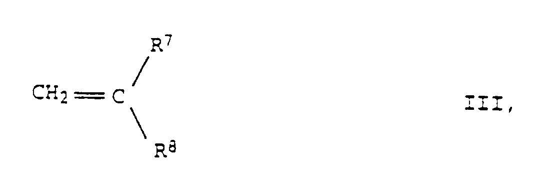 dans laquelle R⁷ est mis pour un atome d'hydrogène ou un reste alkyle en C₁-C₁₀ et R⁸ est mis pour un reste alkyle, alcényle ou aryle.

6. Copolymères modifiés de façon à être utilisables en tant qu'agents dispersants de paraffines, obtenus par réaction de copolymères selon l'une quelconque des revendications 1 à 5, avec des composés à groupements fonctionnels NH , SH et/ou OH.

7. Copolymères modifiés selon la revendication 6, caractérisés en ce que l'on utilise en tant que composé à groupements fonctionnels NH et/ou OH un composé de formule générale Iv 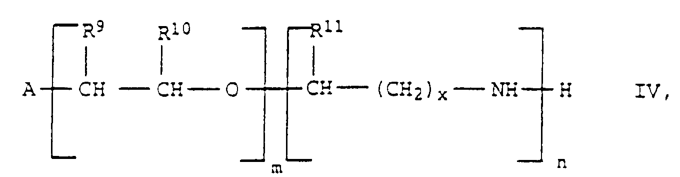 dans laquelle
m peut valoir de 0 à 100, n de 0 à 5 et x de 0 à 5, R⁹, R¹⁰ et R¹¹ sont mis, indépendamment les uns des autres, pour un atome d'hydrogène ou un groupement alkyle en C₁-C₅,
A est mis pour NR¹²R¹³ ou pour un reste alcoxy en C₂-C₃₀, dans lequel au moins un des substituants R¹² ou R¹³ n'est pas un atome d'hydrogène et est mis pour un reste alkyle en C₁-C₃₀ ou alcényle ou un reste polyéther 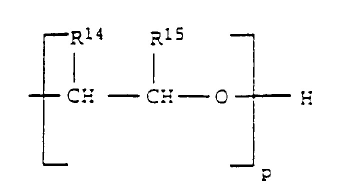 avec R¹⁴ et R¹⁵ mis pour des atomes d'hydrogène ou des groupements alkyle en C₁-C₅ et p vaut de 1 à 100.

8. Utilisation de copolymères selon l'une quelconque des revendications 1 à 7 en tant qu'additifs pour distillats moyens du pétrole.

9. Distillats moyens du pétrole contenant des copolymères selon l'une quelconque des revendications 1 à 7.

10. Procédé de préparation de copolymères selon la revendication 1, caractérisé en ce que l'on fait réagir, en présence d'un amorceur de polymérisation formant des radicaux,
a) 1 à 65 % en moles d'au moins un dicétène de formule générale I, 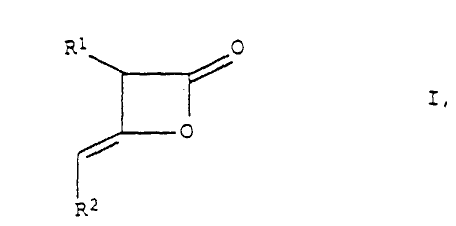 où R¹ et R² sont mis indépendamment l'un de l'autre, pour un atome d'hydrogène ou un groupement alkyle en C₁-C₃₀,
b)30 à 70 % en moles d'au moins un acide dicarboxylique à insaturation éthylénique ou d'un dérivé d'acide dicarboxylique,
c) 0,5 à 60 % en moles d'au moins un hydrocarbure à insaturation éthylénique et
d) jusqu'à 20% en moles d'au moins un autre monomère à insaturation éthylénique.
